# EUROPÄISCHE PATENTSCHRIFT

(11) **EP 0 656 780 B1**
(45) Veröffentlichungstag und Bekanntmachung des Hinweises auf die Patenterteilung: **16.04.1997**
(21) Anmeldenummer: 93919101.1
(22) Anmeldetag: 19.08.1993
(51) Int. Cl.: A61K 31/66, A61K 47/10

(54) **DIPHOSPHONSÄUREN UND DEREN SALZE ENTHALTENDE ARZNEIMITTEL**
DRUGS CONTAINING DIPHOSPHONIC ACIDS AND SALTS THEREOF
MEDICAMENTS CONTENANT DES ACIDES DIPHOSPHONIQUES ET LEURS SELS

(30) Priorität: 27.08.1992 DE 4228552
(43) Veröffentlichungstag der Anmeldung: 14.06.1995
(73) Patentinhaber: BOEHRINGER MANNHEIM GMBH, 68298 Mannheim (DE)
(72) Erfinder: WINTER, Gerhard, D-69221 Dossenheim (DE); PICHLER, Bernhard, D-68775 Ketsch (DE); WOOG, Heinrich, D-69514 Laudenbach (DE); HELLER, Werner, D-67269 Grünstadt (DE)
(86) Internationale Anmeldenummer: EP9302217
(87) Internationale Veröffentlichungsnummer: WO9405297

(56) Entgegenhaltungen:
- EP-A- 0 096 931
- EP-A- 0 252 504
- DATABASE WPI Week 9324, Derwent Publications Ltd., London, GB; AN 93-195043 & SU,A,1 742 242 (LVOV POLY) 23. Juni 1992

## Beschreibung

Die Erfindung betrifft gut verträgliche, in Primärpackmitteln aus Glas lagerstabile Injektionslösungen, die mindestens eine Diphosphonsäure oder mindestens ein physiologisch unbedenkliches Salz einer solchen Säure enthalten, Verfahren zur Herstellung dieser Lösungen und die Verwendung von Polyethylenglykolen zur Stabilisierung dieser Lösungen.

Diphosphonsäuren im Sinne der vorliegenden Erfindung sind Verbindungen, die schon seit einiger Zeit in die therapeutische Praxis zur Behandlung von Kalziumstoffwechselerkrankungen eingeführt sind.

Diese Verbindungen sind insbesondere von Interesse zur Behandlung der Hyperkalzämie und werden eingesetzt als Wirkstoffe in Arzneimitteln zur Behandlung von Osteoporose und bei der Tumorosteolyse. Die Wirksamkeit von Diphosphonaten (Natriumetidronat, Dichlormethylen-diphosphonat, Aminohydroxypropan-, Amino- hydroxybutan-, Aminohydroxypentan-, Aminohydroxyhexandiphosphonat, u.a.) bei der Hemmung der Knochenrückresorption, die auf unnatürliche Weise bei vielen Knochenerkrankungen erhöht ist, wie z. B. bei Morbus Paget, bei Knochentumoren, bei Knochenmetastasen, bei Osteoporose oder bei Hyperparathyreoidismus, ist bereits seit längerer Zeit bekannt.

Diphosphonate als Arzneimittel werden beispielsweise beschrieben in EP 0 170 228; EP 0 197 478; EP 0 224 751; EP 0 252 504; EP 0 252 505; EP 0 258 618 EP 0 350 002; EP 0 273 190; WO 90/00798.

So wird in EP-A-0 096 931 ein lyophilisierter Kit zur Radiodiagnose beschrieben, der Aminodiphosphonate als Radionuclid-Träger, reduzierende Sn²⁺ -Ionen und gegebenenfalls einen Stabilisator enthält. Zur Herstellung des lyophilisierten Kits wird eine wässrige Lösung aus Diphosphonat und Reduktionsmittel hergestellt, und diese Lösung vor der Lyophilisation in Abhängigkeit vom jeweils verwendeten Stabilisator auf einen pH-Wert zwischen 4,2 bis 4,8 oder 5,5 bis 6,5 eingestellt.

Feste Darreichungsformen für diphosphonathaltige Arzneimittel in Form von brausehaltigen Zubereitungen werden beschrieben in DE 3 500 670.

Diphosphonsäuren und ihre Salze sind prinzipiell gut wasserlösliche und gegenüber Temperatureinflüssen in der Regel recht stabile Substanzen. Bei der Herstellung von Injektionslösungen, die auf den pH-Wert des Bluts eingestellt worden waren (pH-Wert 7,4), kam es unerwarteterweise bei längerer Lagerung trotz der guten Löslichkeit der Wirkstoffe zu Trübungen der Lösungen. Daneben hat sich gezeigt, daß sich der Wirkstoffgehalt der Injektionslösungen in den Glasgefäßen bei Lagerung über einen längeren Zeitraum hinweg kontinuierlich erniedrigte, auch wenn die Lösungen noch keine Trübungen zeigten. Die Ursachen für diese Erniedrigung des Wirkstoffgehaltes sind unklar.

In der Regel werden Injektionslösungen in Primärpackmitteln aus Glas (z. B. Ampullen, Vials, Fertigspritzen, Carpulen) abgefüllt, wobei die für pharmazeutische Zwecke eingesetzten Glasampullen üblicherweise aus Gläsern der hydrolytischen Klasse I bestehen. Die chemische Beständigkeit von Glasbehältnissen zur pharmazeutischen Verwendung wird durch Bestimmung von löslichen, mineralischen Substanzen ermittelt, die unter standardisierten Bedingungen an Wasser abgegeben werden. Dabei wird der Kontakt des Wassers mit der Behältnisinnenfläche oder dem gepulverten Glas festgelegt. Die hydrolytische Resistenz wird durch Titration der entstandenen Hydroxidionen in der Lösung bestimmt. Entsprechend ihrer hydrolytischen Resistenz werden die Glasbehältnisse in vier Glasarten eingeteilt. Glas der Klasse I besteht aus Neutralglas mit einer großen hydrolytischen Resistenz aufgrund der chemischen Zusammensetzung des Glases als solches. Glas der Klasse II besteht üblicherweise aus Natronkalk-Silicatglas und besitzt eine große hydrolytische Resistenz, bedingt durch geeignete Oberflächenbehandlung. Glas der Klasse III besteht üblicherweise aus Natronkalk-Silicatglas und besitzt eine mittlere hydrolytische Resistenz. Glas der Klasse IV beseht aus Natronkalk-Silicatglas und besitzt eine geringe hydrolytische Resistenz.

Trotz Verwendung von Glasbehältnissen der hydrolytischen Klasse I mußte festgestellt werden, daß in den Injektionslösungen der Gehalt an Aluminiumionen bei längerer Lagerung stetig anstieg.

Aufgrund dieser drei nachteiligen Befunde bei längerer Lagerung der Lösungen - Trübungen, Erniedrigung des Wirkstoffgehaltes und Erhöhung des Aluminiumgehaltes - mußten derartige Lösungen bezüglich der beabsichtigen behördlichen Zulassung und Registrierung als Arzneimittel als physikalisch instabil gekennzeichnet werden. Außerdem ist es auch aus medizinischer Sicht unerwünscht, wenn Lösungen verabreicht werden, die einen erhöhten Aluminiumgehalt aufweisen.

Aufgabe der Erfindung war es deshalb, in Glasbehältnissen bis zu 5 Jahren lagerstabile Injektionslösungen von Diphosphonsäuren oder ihren Salzen zur Verfügung zu stellen, die bei der Anwendung am Menschen gut verträglich sind.

Es wurde nun gefunden, daß in Glasbehältnissen stabile Injektionslösungen enthaltend als Wirkstoff mindestens eine Diphosphonsäure oder mindestens ein unbedenkliches Salz oder einen Ester einer solchen Säure bereitgestellt werden können, wenn diese Lösungen einen pH-Wert zwischen etwa 3,0 - 4,5 besitzen wobei die Injektionslösungen kein Reduktionsmittel beinhalten oder der pH-Wert etwa 3,0 - 4,5 beträgt und die Lösungen Polyethylenglykole enthalten und gegebenenfalls in Glasbehältnissen abgefüllt sind, die oberflächenvergütet sind.

Überraschenderweise werden bei pH-Werten unter 4,5 Aluminiumkonzentrationen geringer als etwa 2 ppm erreicht, der Wirkstoffgehalt der Lösung bleibt konstant, und es treten keine Trübungen auf. Bevorzugt liegt der pH-Wert der Lösungen bei etwa 4. Die Lösungen sind bis zu fünf Jahren bei Raumtemperatur lagerstabil. Darüber hinaus wurde gefunden, daß die Arzneistofflösung trotz des niedrigen pH-Werts bei intravenöser Gabe gut verträglich und somit für die Anwendung am Menschen gut geeignet ist.

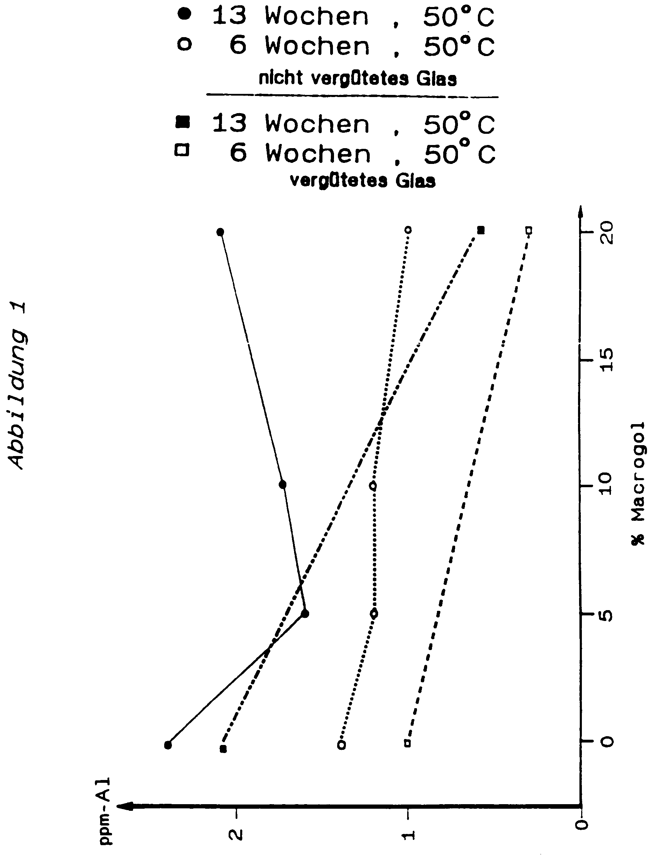

Der Zusatz von Polyethylenglykolen zur Lösung hat den gleichen Effekt wie die pH-Wert-Absenkung. Die verwendbaren Polyethylenglykole besitzen in der Regel ein Molgewicht zwischen 100 und 1500, insbesondere von 200 - 600. Bevorzugt wird Macrogol^{R} eingesetzt. Abbildung 1 verdeutlicht die Verringerung des Aluminiumgehaltes der Lösung bei Zugabe von Macrogol^{R}. Die Menge der zugesetzten Polyethylenglykole kann bis zu 20 Volumenprozent betragen. Bevorzugt setzt man 5 - 10 % der Polyethylenglykole ein. Insbesondere enthält 1 ml Injektionslösung 0,05 bis etwa 0,2 ml Macrogol^{R}.

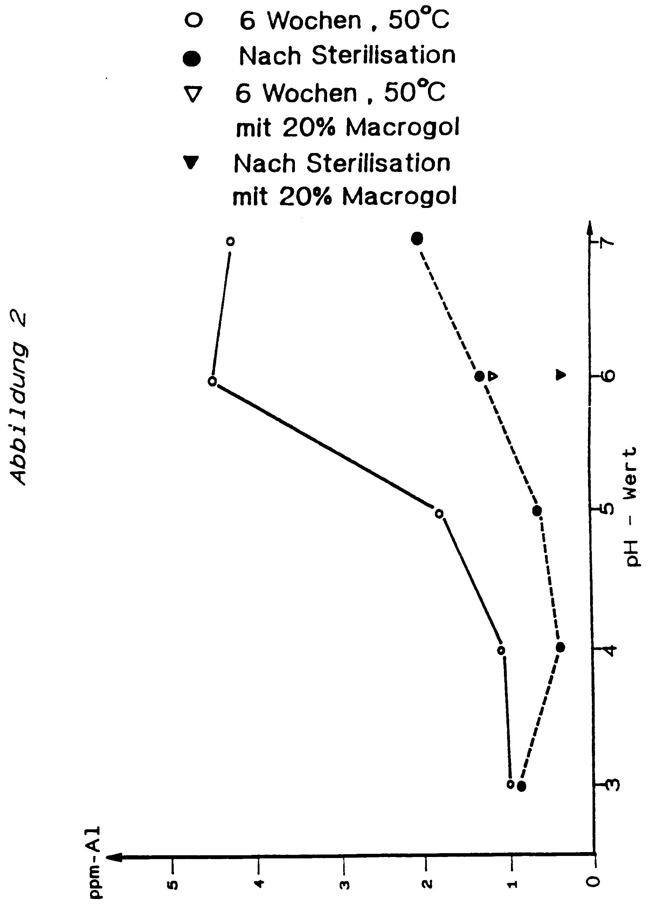

Eine noch stärkere Absenkung des Aluminiumgehaltes wird durch die Kombination beider Maßnahmen - pH-Wert-Absenkung auf etwa 3,0 - 4,5 und Polyethylenglykolzugabe - erreicht (vgl. Abbildung 2).

Die Konzentration der Aluminiumionen kann ferner verringert werden, indem man die Injektionslösung gegebenenfalls unter Inertgasatmosphäre in die Glasampullen abfüllt. Beispielsweise führt eine Begasung der Lösung mit Stickstoff bei ihrer Herstellung zu vergleichbaren Resultaten. Als weitere Maßnahme zur Reduktion des Aluminiumgehaltes kann die Oberfläche des Glasbehältnisses gegebenenfalls vergütet werden. Bei der Vergütung wird eine dünne Oberflächenschicht auf der Innenseite des Ampullenglases erzeugt, indem man beispielsweise Ammoniumsulfat, Schwefeldioxid, Schwefeltrioxid oder Ammoniumchlorid aufdampft.

Im folgenden wird die Erfindung anhand von - zum Teil durch Diagramme illustrierten - Beispielen beschrieben. Als Wirkstoffe werden stellvertretend für die Gruppe der Diphosphonate 1-Hydroxy-3-(N-methyl-N-pentyl)aminopropyl-1,1-diphosphonsäure (=A), Dichlor-methan-diphosphonsäure (=B, Clodronat) bzw. 3-Amino-1-Hydroxypropyliden-diphosphonsäure (=C, Pamidronat) in einer für die Injektion geeigneten Lösung eingesetzt. Der Wirkstoff A ist in der Europäischen Anmeldung EP 0 252 505 beschrieben. Prinzipiell können auch andere Diphosphonsäuren oder deren physiologisch unbedenklichen Salze oder deren physiologisch unbedenklichen Ester in entsprechender Weise eingesetzt werden.

Die Abbildungen 1 und 2 zeigen in Diagrammen den Aluminiumgehalt der in den Beispielen 9 bis 15 beschriebenen Injektionslösungen in Abhängigkeit von den Faktoren pH-Wert, Hilfsstoffzusatz und Lagerzeit.

### Beispiele 1 bis 9

Die in den Beispielen 1 bis 9 beschriebenen Injektionslösungen enthielten pro ml wässriger Lösung jeweils 1,069 mg Natriumsalz der 1-Hydroxy-3-(N-methyl-N-pentyl)aminopropyl-1,1-diphosphonsäure und 8,6 mg NaCl. Die Bestandteile bzw. Eigenschaften, die nicht bei allen Lösungen identisch waren, sind in der Tabelle 1 aufgeführt. Die Einstellung der pH-Werte erfolgte durch die Zu-gabe von entsprechenden Mengen NaOH bzw. HCl. Die Lösungen wur-den in Ampullen aus Glas der hydrolytischen Klasse 1 eingefüllt. Die Lösungen wurden vor dem Lagern 20 Minuten lang bei 121°C sterilisiert. Vor und nach dem Sterilisieren und nach einer Lagerzeit von sechs Wochen bei 50°C wurde der Aluminiumgehalt in den Lösungen mittels AAS (Atomabsorptionsspektroskopie) bestimmt. Die Aluminiumwerte - gemittelt über jeweils zehn Lösungsproben pro Beispiel - sind ebenfalls in der Tabelle 1 aufgelistet.

**Tabelle 1**

| Bsp. Nr. | pH | N2-Begasen | Macrogol [ml/ml Ls.] | Al-Gehalt [ppm] | | nach 6 Wochen |
|---|---|---|---|---|---|---|
| | | | | vor Steril. | nach Steril. | |
| 1 | 3 | | | 0,4 | 0,9 | 1,0 |
| 2 | 4 | | | 0,2 | 0,4 | 1,1 |
| 3 | 4,5 | | | 0,2 | 0,5 | 1,2 |
| 4 | 5 | | | 0,3 | 0,6 | 1,8 |
| 5 | 6 | | | 0,6 | 1,3 | 4,5 |
| 6 | 6 | + | | 0,4 | 0,8 | 4,6 |
| 7 | 6 | | 0,2 | 0,3 | 0,4 | 1,2 |
| 8 | 6 | | | 0,3 | 0,7 | 4,4 1) |
| 9 | 7 | | | 1,2 | 2,1 | 4,3 |

| | | | | | | |
|---|---|---|---|---|---|---|
| 1) Zusatz von Propylenglykol (0,2ml pro 1ml Lösung) | | | | | | |

Die Ergebnisse zeigen, daß niedrige pH-Werte und ein Zusatz von Polyethylenglykol, wie z.B. Macrogol, (s. Bsp. 7) den Aluminiumgehalt besonders niedrig zu halten vermögen, daß die N2-Begasung bei der Herstellung der Lösung wenigstens anfänglich zu einem erniedrigten Aluminiumgehalt der Lösungen beiträgt und daß zwar die Zugabe von anderen organischen, Hydroxylgruppen aufweisenden Lösungsmitteln auch den Aluminiumgehalt in der Lösung vermindert (s. Bsp. 8), aber wesentlich weniger als Polyethylenglykole.

### Beispiele 10 bis 16

Die in den Beispielen 10 bis 16 beschriebenen Injektionslösungen enthielten pro ml wässriger Lösung jeweils 1,12 mg Natriumsalz der 1-Hydroxy-3-(N-methyl-N-pentyl)aminopropyl-1,1-diphosphonsäure und 8,6 mg NaCl. Bei der Herstellung wurden die Lösungen mit Stickstoff begast. Die Bestandteile bzw. Eigenschaften, die nicht bei allen Lösungen identisch waren, sind in der Tabelle 2 aufgeführt. Die Einstellung des pH-Werts beim Beispiel 16 erfolgte durch die Zugabe einer entsprechenden Menge NaOH. Die Lösungen der Beispiele 10 bis 13 wurden in Ampullen aus Glas der hydrolytischen Klasse 1 und die der Lösungen 14 bis 16 in OPC-Ampullen mit Ammoniumsulfat vergüteter Oberfläche eingefüllt. Die Lösungen wurden vor dein Lagern 20 Minuten lang bei 121°C sterilisiert. Nach dein Sterilisieren und nach Lagerzeiten von 6 und 13 Wochen bei 50°C wurde der Aluminiumgehalt in den Lösungen mittels AAS bestimmt. Die Aluminiumwerte - gemittelt über jeweils zehn Lösungsproben pro Beispiel - sind ebenfalls in der Tabelle 2 aufgelistet.

**Tabelle 2**

| Bsp. Nr. | Glas vergütet | Macrogol [ml/ml Ls.] | pH | Al-Gehalt [ppm] | | nach 13 Wochen |
|---|---|---|---|---|---|---|
| | | | | Steril. | 6 Wochen | |
| 10 | | | 4 | 0,5 | 1,4 | 2,4 |
| 11 | | 0,05 | 4 | 0,6 | 1,2 | 1,6 |
| 12 | | 0,10 | 4 | 0,7 | 1,2 | 1,7 |
| 13 | | 0,20 | 4 | 0,6 | 1,0 | 2,1 |
| 14 | + | | 4 | 0,1 | 1,0 | 2,1 |
| 15 | + | 0,20 | 4 | 0,1 | 0,3 | 0,6 |
| 16 | + | | 6 | 0,8 | 4,5 | 5,9 |

In den Abbildungen 1 und 2 sind über der Lagerungszeit die Aluminiumwerte der in den Beispielen 11 bis 13 bzw. 14 bis 16 beschriebenen erfindungsgemäßen Lösungen und zum Vergleich die Aluminiumwerte der im Beispiel 10 beschriebenen Lösung aufgetragen, die als erfindungsgemäßes Merkmal zur Verminderung des Aluminiumgehalts in den Injektionslösungen nur die - bei allen Beispielen der zweiten Serie angewandte - N₂-Begasung aufweist.

Die Ergebnisse zeigen - analog den bei der ersten Serie von Beispielen erzielten -, daß Macrogol den Aluminiumgehalt beachtlich erniedrigt (s. Bsp. 11 -13), wobei sogar kleinere Mengen effektiver zu sein scheinen als größere. Sie zeigen außerdem, daß vergütetes Glas allein (s. Bsp. 14) oder zusammen mit Macrogol (s. Bsp. 15) eine Barriere gegen das Herauslösen des Aluminiums bildet, aber auch daß die Wirkung eines hohen pH-Werts nicht durch vergütetes Glas abgeblockt werden kann (s. Bsp. 16).

### Beispiel 17

### Untersuchungen zur Haltbarkeit der Lösungen in Abhängigkeit des pH-Wertes der Lösungen

Zur Herstellung der Injektionslösungen wurden die folgenden Wirkstoffe verwendet:
- A =: 1-Hydroxy-3-(N-methyl-N-pentyl)aminopropyl-1,1-diphosphonsäure; Konzentration: 1 mg/ml
- B =: 1,1-Dichlor-1,1-diphosphonsäure, Na-Salz x 4 H₂O; Konzentration: 300 mg/10 ml
- C =: 3-Amino-1-Hydroxypropyliden-diphosphonsäure; Konzentration: 3 mg/1 ml

Die Aluminiumbestimmungen erfolgten durch AAS. Die angegebenen Daten sind Mittelwerte aus fünf bzw. zehn Bestimmungen. Die Herstellung der Injektionslösungen erfolgte nach der folgenden Vorschrift: Der Wirkstoff wird in Wasser für Injektionszwecke gelöst, und mit NaOH oder HCl der gewünschte pH-Wert eingestellt. Im Fall des Wirkstoffes B erfolgte die Einstellung des gewünschten pH-Wertes mit Natriumhydrogencarbonat. Die Abfüllung in Ampullen erfolgt nach Filtration über sterilisierte Membranfilter der Porenweite 0,2 µm unter aseptischen Bedingungen. Lösungen mit Wirkstoff A und C wurden zusätzlich bei 121 °C sterilisiert. Eingesetzt werden Glasampullen der hydrolytischen Klasse I.

**Tabelle 3**

| Wirkstoff | pH | Temp. [°C] | Lagerungszeit (Mon.) | Al-Gehalt [ppm] |
|---|---|---|---|---|
| - | 6 | 21-25 | 19 | 0,3 |
| A | 6 | 21-25 | 8 | 3,2 |
| A | 6 | 50 | 1,5 | 4,4 |
| A | 5 | 50 | 1,5 | 1,8 |
| A | 4 | 50 | 1,5 | 1,1 |
| A | 4 | 50 | 3 | 1,3 |
| A | 4 | 30 | 3 | 0,7 |
| A | 3 | 50 | 1,5 | 1,0 |
| B | 5,7 | 25 | 22 | 3,6 |
| B | 4,3 | 25 | 21 | 0,6 |
| C | 4,3 | 50 | 3 | 1,9 |
| C | 6,5 | 50 | 3 | 3,9 |

Wie der obigen Tabelle zu entnehmen ist, erhöht sich der Aluminiumgehalt der Injektionslösung, wenn die Lösungen einen pH-Wert aufweisen, der in der Nähe des pH-Wertes des Blutes liegt und die Lösungen unter Belastung gelagert werden. Die stärkere Belastung der Proben kann erreicht werden durch längere Lagerung bei Raumtemperatur oder durch Lagerung bei erhöhten Temperaturen über einen entsprechend kürzeren Zeitraum hinweg. Lösungen, die einen pH-Wert unter 5 aufweisen, besitzen einen vergleichsweise geringeren Aluminiumgehalt.

### Beispiel 18

Die in Beispiel 18 beschriebenen Injektionslösungen enthalten pro ml wäßriger Lösung jeweils 3 mg Pamidronat = APD = 3-Amino-Hydroxypropyliden-Bisphosphonsäure. Bei der Herstellung der Lösungen wurde mit kleinen Mengen NaOH der pH-Wert auf 4, 5, 6 eingestellt. Jeweils 1 ml der Lösungen wurde in 1 ml-Glasampullen der hydrolytischen Klasse 1 abgefüllt. Die Ampullen wurden bei Raumtemperatur (21 - 25°C) sowie 50°C gelagert und dann wurde mittels AAS der Aluminiumgehalt in den Lösungen bestimmt. In Tabelle 4 sind die Mittelwerte aus jeweils 5 Messungen in Abhängigkeit von Lagertemperatur und pH angegeben. Aus den Daten geht klar hervor, daß die Absenkung des pH-Wertes von 6 auf 5 bzw. 4 die Aluminiumgehalte auf ca. 50 % senkt.

**Tabelle 4**

| Aluminiumionen in Glasampullen mit Pamidronat (APD) 3 mg/1 ml-Injektionslösung | | | |
|---|---|---|---|
| pH-Wert der Injektionslösung | Lagerungsdauer (Wochen) | Temperaturbelastung | Al-Gehalt (ppm) pro Ampulle Mittelwert aus aus 5 Messungen |
| 4 | 8 | RT | 0,46 |
| 4 | 8 | RT | 0,46 |
| 4 | 8 | 50 °C | 0,72 |
| 4 | 8 | 50 °C | 0,84 |
| 5 | 8 | RT | 0,54 |
| 5 | 8 | RT | 0,47 |
| 5 | 8 | 50 °C | 1,00 |
| 5 | 8 | 50 °C | 0,65 |
| 6 | 8 | RT | 0,80 |
| 6 | 8 | RT | 0,75 |
| 6 | 8 | 50 °C | 1,68 |
| 6 | 8 | 50 °C | 1,40 |
| RT=Raumtemperatur | | | |

### Beispiel 19

Die in Beispiel 19 beschriebenen Injektionslösungen enthalten pro ml wässriger Lösung jeweils 1,069 mg Natriumsalz der 1-Hydroxy-3-(N-methyl-N-pentyl)aminopropyl-1,1-diphosphonsäure und 8,6 mg NaCl. Der pH-Wert wurde mit 0,5 mg Essigsäure und 0,2 mg Natriumacetat (3H₂O) eingestellt. Die Lösungen wurden in Ampullen aus Glas der hydrolytischen Klasse I eingefüllt und vor der Lagerung 20 Minuten lang bei 121 °C sterilisiert.

Nach entsprechender Lagerdauer wurde der Aluminiumgehalt in Lösung mittels AAS bestimmt. Die Ergebnisse sind in Tabelle 5 dargestellt. Alle Lösungen waren nach der Lagerung klar und wiesen den vollen Wirkstoffgehalt auf. Die Verträglichkeit dieser Zubereitungen nach i.v.-Injektion war sehr gut.

| Injektionslösung Nr. | Lagerungsdauer (Wochen) | Temperaturbelastung °C | pH-Wert d.Injektionslösung | Al-Gehalt (ppm) pro Ampulle Mittelwert aus aus 5 Messungen |
|---|---|---|---|---|
| 19.1 | 52 | 6 - 8 | 3,9 | 0,47 |
| 19.1 | 52 | 25 | 3,9 | 0,6 |
| 19.1 | 52 | 30 | 4,0 | 0,73 |
| 19.2 | 52 | 6 - 8 | 4,3 | 0,58 |
| 19.2 | 52 | 25 | 4,3 | 1,0 |
| 19.2 | 52 | 30 | 4,3 | 1,18 |
| 19.3 | 52 | 6 - 8 | 4,1 | 0,13 |
| 19.3 | 52 | 25 | 4,1 | 0,16 |
| 19.3 | 52 | 30 | 4,1 | 0,28 |
| 19.4 | 52 | 6 - 8 | 4,3 | 0,39 |
| 19.4 | 52 | 25 | 4,3 | 0,63 |
| 19.4 | 52 | 30 | 4,3 | 0,77 |

## Patentansprüche

1. Lagerstabile Injektionslösungen enthaltend als Wirkstoff mindestens eine Diphosphonsäure oder mindestens ein physiologisch unbedenkliches Salz oder einen Ester einer solchen Säure, dadurch gekennzeichnet, daß der pH-Wert der Injektionslösung 3,0 - 4,5 beträgt, wobei die Injektionslösung kein Reduktionsmittel beinhaltet, oder der pH-Wert 3,0 - 4,5 beträgt und die Lösung Polyethylenglykole enthält.

2. Injektionslösung nach Anspruch 1, dadurch gekennzeichnet, daß der pH-Wert der Lösung weniger als 4,5 beträgt und die Lösung Polyethylenglykole enthält.

3. Injektionslösung nach Anspruch 1 oder 2, dadurch gekennzeichnet, daß der pH-Wert etwa 4 beträgt.

4. Injektionslösung nach einem der Ansprüche 1 bis 3, dadurch gekennzeichnet, daß die Lösung bis zu 20 % Polyethylenglykole mit einer Molmasse von 200 - 1500 enthält.

5. Injektionslösung nach Anspruch 4, dadurch gekennzeichnet, daß die Lösung 0,05 bis 0,2 ml Macrogol pro 1 ml Injektionslösung enthält.

6. Injektionslösung nach einem der Ansprüche 1 bis 5, dadurch gekennzeichnet, daß die Lösung als Wirkstoff mindestens eine 1-Hydroxy-aminoalkyl-1,1-diphosphonsäure und/oder mindestens ein physiologisch unbedenkliches Salz oder einen physiologisch unbedenklichen Ester einer solchen Säure enthält.

7. Injektionslösung nach Anspruch 6, dadurch gekennzeichnet, daß die Lösung als Wirkstoff 1-Hydroxy-3-(N-methyl-N-pentyl)aminopropyl-1,1-diphosphonsäure oder eines ihrer physiologisch unbedenklichen Salze enthält.

8. Injektionslösung nach einem der Ansprüche 1 bis 7, dadurch gekennzeichnet, daß der Wirkstoff als Alkalisalz vorliegt.

9. Injektionslösung nach einem der Ansprüche 1 bis 8, dadurch gekennzeichnet, daß die Lösung den Wirkstoff in einer Menge zwischen 0,1 und 1000 mg und NaCl in einer Menge von weniger als 10 mg/ml wässriger Lösung enthält.

10. Verfahren zur Herstellung von lagerstabilen Injektionslösungen enthaltend als Wirkstoff mindestens eine Diphosphonsäure oder mindestens ein physiologisch unbedenkliches Salz oder einen Ester einer solchen Säure, dadurch gekennzeichnet, daß man den pH-Wert der wirkstoffhaltigen pharmazeutischen Lösung vor dem Abfüllen in Primärpackmittel aus Glas auf 3,0 - 4,5 einstellt, der Injektionslösung jedoch kein Reduktionsmittel zusetzt, oder den pH-Wert auf 3,0 - 4,5 einstellt und Polyethylenglykole zusetzt und gegebenenfalls in Glasbehältnisse abfüllt, die oberflächenvergütet sind.

11. Verfahren zur Herstellung gemäß Anspruch 10, dadurch gekennzeichnet, daß man in oberflächenvergütete Glasbehältnisse abfüllt, die mit Ammoniumsulfat, Schwefeldioxid, Schwefeltrioxid oder Ammoniumchlorid vergütet sind.

12. Verfahren gemäß Anspruch 10 oder 11, dadurch gekennzeichnet, daß der pH-Wert auf etwa 4 eingestellt wird.

13. Verwendung von Polyethylenglykolen zur Stabilisierung von Injektionslösungen in Glasbehältnissen enthaltend als Wirkstoft mindestens eine Diphosphonsäure oder mindestens ein physiologisch unbedenkliches Salz oder einen Ester einer solchen Säure.

## Claims

1. Injection solutions that are stable on storage containing at least one diphosphonic acid or at least one physiologically acceptable salt or one ester of such an acid as the active substance, wherein the pH value of the injection solution is 3.0 - 4.5, the injection solution comprising no reducing agent or the pH value is 3.0 - 4.5 and the solution contains polyethylene glycols.

2. Injection solution as claimed in claim 1, wherein the pH value of the solution is less than 4.5 and the solution contains polyethylene glycols.

3. Injection solution as claimed in claim 1 or 2, wherein the pH value is about 4.

4. Injection solution as claimed in one of the claims 1 to 3, wherein the solution contains up to 20 % polyethylene glycols having a molar mass of 200 - 1500.

5. Injection solution as claimed in claim 4, wherein the solution contains 0.05 to 0.2 ml Macrogol per 1 ml injection solution.

6. Injection solution as claimed in one of the claims 1 to 5, wherein the solution contains at least one 1-hydroxy-aminoalkyl-1,1-diphosphonic acid and/or at least one physiologically acceptable salt or one physiologically acceptable ester of such an acid as the active substance.

7. Injection solution as claimed in claim 6, wherein the solution contains 1-hydroxy-3-(N-methyl-N-pentyl)aminopropyl-1,1-diphosphonic acid or one of its physiologically acceptable salts as the active substance.

8. Injection solution as claimed in one of the claims 1 to 7, wherein the active substance is present as an alkali salt.

9. Injection solution as claimed in one of the claims 1 to 8, wherein the solution contains the active substance in an amount between 0.1 and 1000 mg and NaCl in an amount of less than 10 mg/ml aqueous solution.

10. Process for the production of injection solutions that are stable on storage containing at least one diphosphonic acid or at least one physiologically acceptable salt or one ester of such an acid as the active substance, wherein the pH value of the pharmaceutical solution containing the active substance is adjusted to 3.0 - 4.5 before being filled into the primary packaging made of glass, however, no reducing agent is added to the injection solution or the pH value is adjusted to 3.0 - 4.5 and polyethylene glycols are added and, if desired, it is filled into glass containers which are surface-treated.

11. Process for the production as claimed in claim 10, wherein it is filled into surface-treated glass containers which have been treated with ammonium sulfate, sulphur dioxide, sulphur trioxide or ammonium chloride.

12. Process as claimed in claim 10 or 11, wherein the pH value is adjusted to about 4.

13. Use of polyethylene glycols to stabilize injection solutions in glass containers containing at least one diphosphonic acid or at least one physiologically acceptable salt or one ester of such an acid as the active substance.

## Revendications

1. Solutions injectables stables au stockage, contenant, en tant que principe actif, au moins un acide diphosphonique ou au moins un sel ou ester physiologiquement acceptable d'un tel acide, caractérisées en ce que le pH de la solution injectable est de 3,0-4,5 alors que la solution injectable ne contient pas d'agent réducteur, ou le pH est de 3,0-4,5 et la solution contient du polyéthylèneglycol.

2. Solution injectable selon la revendication 1, caractérisée en ce que le pH de la solution est inférieure à 4,5 et que la solution contient du polyéthylèneglycol.

3. Solution injectable selon la revendication 1 ou 2, caractérisée en ce que le pH est d'environ 4.

4. Solution injectable selon l'une quelconque des revendications 1 à 3, caractérisée en ce que la solution contient jusqu'à 20 % de polyéthylèneglycol ayant une masse moléculaire de 200-1500.

5. Solution injectable selon la revendication 4, caractérisée en ce que la solution contient de 0,05 à 0,2 ml de Macrogol pour 1 ml de solution injectable.

6. Solution injectable selon l'une quelconque des revendications 1 à 5, caractérisée en ce que la solution contient, en tant que principe actif, au moins un acide 1-hydroxy-aminoalkyl-1,1-diphosphonique et/ou au moins un sel physiologiquement acceptable ou un ester physiologiquement acceptable d'un tel acide.

7. Solution injectable selon la revendication 6, caractérisée en ce que la solution contient, en tant que principe actif, l'acide 1-hydroxy-3-(N-méthyl-N-pentyl)aminopropyl-1,1-diphosphonique ou un de ses sels physiologiquement acceptables.

8. Solution injectable selon l'une quelconque des revendications 1 à 7, caractérisée en ce que le principe actif est présent sous forme de sel alcalin.

9. Solution injectable selon l'une quelconque des revendications 1 à 8, caractérisée en ce que la solution contient le principe actif en une quantité comprise entre 0,1 et 1000 mg et NaCl en une quantité inférieure à 10 mg/l de solution aqueuse.

10. Procédé de préparation de solutions injectables stables au stockage, contenant, en tant que principe actif, au moins un acide diphosphonique ou au moins un sel ou ester physiologiquement acceptable d'un tel acide, caractérisé en ce que l'on ajuste à 3,0-4,5 le pH de la solution pharmaceutique contenant le principe actif avant de conditionner cette solution dans des emballages primaires en verre, toutefois, on n'ajoute pas d'agent réducteur à la solution injectable, ou on ajuste le pH à 3,0-4,5 et on ajoute du polyéthylèneglycol et éventuellement, on conditionne la solution dans des récipients en verre qui ont subi un traitement de surface.

11. Procédé de préparation selon la revendication 10, caractérisé en ce que l'on conditionne la solution dans des récipients en verre ayant subi un traitement de surface, qui ont été traités avec du sulfate d'ammonium, du dioxyde de soufre, du trioxyde de soufre ou du chlorure d'ammonium.

12. Procédé selon la revendication 10 ou 11, caractérisé en ce que l'on ajuste le pH à environ 4.

13. Utilisation de polyéthylèneglycols pour la stabilisation de solutions injectables dans des récipients en verre, contenant, en tant que principe actif, au moins un acide diphosphonique ou au moins un sel ou ester physiologiquement acceptable d'un tel acide.
